# FASCICULE DE BREVET EUROPEEN

(11) **EP 3 881 069 B1**
(45) Date de publication et mention de la délivrance du brevet: **21.06.2023**
(21) Numéro de dépôt: 19797257.3
(22) Date de dépôt: 30.10.2019
(51) Int. Cl.: G01N 33/28, G01N 21/51

(54) **PROCEDE DE DETERMINATION D'AU MOINS UN PARAMETRE REPRESENTATIF D'UN CHANGEMENT D'UN FLUIDE PAR SPECTROSCOPIE PROCHE INFRAROUGE MULTIPOINTS**
VERFAHREN ZUR BESTIMMUNG MINDESTENS EINES EINE VERÄNDERUNG IN EINEM FLUID DARSTELLENDEN PARAMETERS DURCH MULTIPUNKT-NAHINFRAROTSPEKTROSKOPIE
METHOD FOR DETERMINING AT LEAST ONE PARAMETER THAT REPRESENTS A CHANGE IN A FLUID BY MULTI-POINT NEAR-INFRARED SPECTROSCOPY

(30) Priorité: 16.11.2018 FR 1871571
(43) Date de publication de la demande: 22.09.2021
(73) Titulaire: IFP Energies nouvelles, 92852 Rueil-Malmaison Cedex (FR)
(72) Inventeur: REY-BAYLE, Maud, 92852 Ruel-Malmaison Cedex (FR); CAILLOL, Noemie, 92852 Ruel-Malmaison Cedex (FR); CASSAR, Cyril, 92852 Ruel-Malmaison Cedex (FR); DARBOURET, Myriam, 92852 Ruel-Malmaison Cedex (FR)
(74) Mandataire: IFP Energies nouvelles
(86) Numéro de dépôt international: PCT/EP2019/079625
(87) Numéro de publication internationale: WO 2020/099130

(56) Documents cités:
- WO-A1-2016/209248
- FR-A1- 2 647 903
- US-A1- 2016 097 717
- US-A1- 2016 208 601
- K. PASO ET AL: "Measurement of Wax Appearance Temperature Using Near-Infrared (NIR) Scattering", ENERGY & FUELS., vol. 23, no. 10, 15 octobre 2009 (2009-10-15), pages 4988-4994, XP055611856, WASHINGTON, DC, US. ISSN: 0887-0624, DOI: 10.1021/ef900173b
- TUNE B. BONN? ET AL: "Aggregation behavior of amphiphilic poly(2-alkyl-2-oxazoline) diblock copolymers in aqueous solution studied by fluorescence correlation spectroscopy", COLLOID & POLYMER SCIENCE, vol. 282, no. 8, 1 juin 2004 (2004-06-01), pages 833-843, XP055240664, DE ISSN: 0303-402X, DOI: 10.1007/s00396-004-1131-2
- MORITA S ET AL: "Computational simulations and a practical application of moving-window two-dimensional correlation spectroscopy", JOURNAL OF MOLECULAR STRUCTURE, ELSEVIER, AMSTERDAM, NL, vol. 799, no. 1-3, 6 novembre 2006 (2006-11-06), pages 111-120, XP028046052, ISSN: 0022-2860, DOI: 10.1016/J.MOLSTRUC.2006.03.023 [extrait le 2006-11-06]

## Description

### Domaine technique

La présente invention concerne le domaine de la caractérisation d'un fluide, en particulier un brut pétrolier. La caractérisation du fluide détermine au moins un paramètre du fluide, le paramètre étant lié à un changement (c'est-à-dire une transition) du fluide dû à une variation de température du fluide. En particulier, l'invention concerne la détermination de la température de cristallisation commençante de paraffine (Tcc) ou WAT (de l'anglais « Wax Apparition Température ») dans le fluide et/ou la température du point d'écoulement du fluide PP (de l'anglais « Pour Point »).

Cette invention s'inscrit notamment dans le cadre des besoins de caractérisation et de compréhension des propriétés à froid des produits pétroliers pour le raffinage et dans le domaine de l'exploration-production.

### Technique antérieure

Dans ce cadre, les propriétés à froid sont, entres autres, caractérisées par la température d'apparition de paraffine WAT, et le point d'écoulement PP. De manière classique, les caractérisations de ces deux propriétés sont normalisées, par calorimétrie différentielle à balayage DSC (en anglais « Differential Scanning Calorimetry ») pour la WAT, et par une mesure physique via l'inclinaison d'un récipient à différentes températures pour la PP. Ces méthodes sont longues, peuvent s'avérer imprécises, et ne peuvent pas se faire en ligne en conditions d'écoulement du produit pétrolier. Des approches indirectes ont été développées mais elles ne donnent accès que partiellement au comportement à froid des produits. En outre, la plupart des solutions commerciales ne sont pas adaptées à des produits opaques voire noirs comme peuvent l'être les bruts pétroliers.

La détermination des propriétés à froid dans le domaine pétrolier n'est pas un sujet de recherche nouveau, de nombreuses méthodes ont été développées.

Une première solution, décrite notamment dans la demande de brevet US 5454257 consiste à déterminer l'apparition de paraffines à partir de la modification du volume de la cellule où est le produit pétrolier en fonction de la température. Une telle méthode nécessite un échantillon de fluide et ne peut pas être utilisée en ligne en continu.

Une autre méthode, décrite notamment dans la demande de brevet US2016/0208601, porte sur la détermination de la WAT en déterminant la température à laquelle la pression n'est plus stable dans un système micro-fluidique où circule le produit. Une telle méthode nécessite également un échantillon de fluide et ne peut pas être utilisée pour un suivi en ligne d'une circulation continue d'un fluide .

Il existe également des solutions qui utilisent des mesures sur le milieu pour déterminer des propriétés à froid en s'appuyant sur les interactions de la lumière avec la matière. Plusieurs travaux utilisent la spectroscopie proche infrarouge en transflectance. Le document "Paso K, Kallevik H, Sjöblom J. Measurement of Wax Appearance Température Using Near-Infrared (NIR) Scattering. Energy Fuels 2009; doi:10.1021/ef900173b" décrit une détermination de la WAT sur plusieurs fluides pétroliers (huiles brutes, waxy gas condensate, et des échantillons de macrocrystalline et microcrystalline paraffin wax dans du dodecane). Les auteurs suivent à quelques longueurs d'onde la densité optique de la lumière diffusée. Aucune analyse multivariée des mesures spectrales n'a été mise en oeuvre pour déterminer précisément la WAT.

Le document "Santos D, Filho EBM, Dourado RS, Amaral M, Filipakis S, Oliveira, Lize M. S. L., Guimarâes RCL, Santos AF, Borges GR, Franceschi E, Dariva C. Study of Asphaltene Précipitation in Crude Oils at Desalter Conditions by Near-Infrared Spectroscopy. Energy Fuels 2017; doi:10.1021/acs.energyfuels.7b00602" illustre, quant à lui, la détermination de la précipitation des asphaltènes dans un brut, au moyen d'une mesure en un unique point.

Une autre invention, telle que décrite notamment dans la demande de brevet US2003/0075478, propose également de déterminer la WAT en ligne grâce à la réflexion d'une source laser.

De plus, une autre méthode, décrite notamment dans la demande de brevet US2016/0097717, porte sur un système permettant de déterminer la température d'apparition et de disparition des cristaux de paraffines dans des huiles transparentes, translucides et opaques en mesurant le signal diffusé en un point en réflexion. Le système permet la circulation et la chauffe du fluide. Une telle méthode nécessite un échantillon de fluide et ne peut pas être utilisée pour un suivi en ligne d'une circulation continue d'un fluide.

En outre, il est également intéressant de déterminer d'autres paramètres représentatifs de changements d'un fluide. Il peut s'agir notamment de paramètres liés à un seuil de transition entre les phases du fluide, un point d'apparition des cristaux, un paramètre lié à un agrégat ou une agglomération d'objets au sein du fluide, par exemple la génération de nano-agrégats.

### Résumé de l'invention

Pour pallier ces inconvénients, la présente invention concerne un procédé de détermination d'au moins un paramètre représentatif d'un changement d'un fluide dû à une variation de température du fluide, pour lequel on dispose le fluide dans un milieu transparent au domaine proche infrarouge et on réalise une mesure spectrale par spectroscopie proche infrarouge multi points (également appelée spectroscopie proche infrarouge résolue spatialement) et multi températures. Ensuite, par analyse multivariée de la mesure on en déduit le paramètre recherché. Le procédé selon l'invention est adapté à tout type de fluide, y compris les fluides opaques, grâce à la spectroscopie résolue spatialement, et permet de réaliser rapidement et simplement des mesures en ligne pour un suivi d'une circulation continue d'un fluide grâce à la spectroscopie proche infrarouge. De plus, La réalisation d'une spectroscopie multipoints et multi températures permet d'obtenir précisément le paramètre recherché, et, le cas échéant, de déterminer plusieurs paramètres du fluide. En effet, l'analyse multivariée permet de déterminer plusieurs caractéristiques du fluide.

En outre, l'invention concerne un procédé de surveillance et/ou de contrôle d'un écoulement d'un fluide dans une conduite en mettant en oeuvre un tel procédé de détermination d'un paramètre représentatif d'un changement du fluide.

L'invention concerne un procédé de détermination d'au moins un paramètre représentatif d'un changement d'un fluide dû à une variation de température dudit fluide. Pour ce procédé, on met en oeuvre les étapes suivantes :
a) on dispose ledit fluide dans un milieu transparent au domaine proche infrarouge ;
b) on réalise une mesure spectrale par spectroscopie proche infrarouge résolue spatialement pour ledit fluide disposé dans ledit milieu transparent, ladite mesure spectrale étant réalisée pour au moins deux angles de mesure et pour au moins deux températures dudit fluide ;
c) on réalise une analyse multivariée de ladite mesure spectrale en fonction de ladite température ; et
d) on détermine ledit paramètre représentatif d'un changement dudit fluide au moyen de ladite analyse multivariée.

Selon un mode de réalisation, ledit paramètre représentatif d'un changement dudit fluide est choisi parmi la température d'apparition de paraffine dans ledit fluide, la température du point d'écoulement dudit fluide, un point d'apparition des cristaux, une température limite de filtrabilité, un seuil de transition entre les phases dudit fluide, un paramètre lié à un agrégat ou une agglomération d'objets au sein dudit fluide, tel que la génération de nano-agrégats, la croissance des objets, la poly-dispersité des objets.

De préférence, ledit paramètre représentatif d'un changement dudit fluide est la température d'apparition de paraffine dans ledit fluide et/ou la température du point d'écoulement dudit fluide.

Avantageusement, ledit fluide est un brut pétrolier.

Selon un aspect, on dispose ledit fluide dans une déviation d'un conduit d'écoulement dudit fluide.

Conformément à une mise en oeuvre, on réalise ladite spectroscopie proche infrarouge résolue spatialement au moyen d'au moins une mesure en transmission, notamment avec un angle de mesure (α) compris entre 130°et 180°, de préférence entre 1 65°et 180°.

Avantageusement, on réalise ladite spectroscopie proche infrarouge résolue spatialement au moyen d'au moins une mesure en réflexion, notamment avec un angle de mesure (α) compris entre 5°et 90°, de préférence entre 20 e t 40°.

De manière avantageuse, on réalise ladite spectroscopie proche infrarouge résolue spatialement au moyen d'au moins deux mesures en transmission avec respectivement des angles de mesure (α) choisis parmi environ 170°, 175°et 180°et au mo yen d'une mesure en réflexion avec un angle de mesure (α) de environ 30°.

Selon une caractéristique, ladite analyse multivariée est une analyse en composantes principales ou une analyse en composantes communes et poids spécifiques.

De préférence, ladite analyse multivariée est réalisée pour au moins six composantes.

Avantageusement, on détermine ledit paramètre représentatif d'un changement dudit fluide par analyse d'au moins un point d'inflexion et/ou une rupture de pente et/ou la limitation du bruit d'au moins une composante de ladite analyse multivariée.

Conformément à un mode de réalisation, ladite spectroscopie proche infrarouge résolue spatialement est mise en oeuvre avec variation de la longueur d'onde entre une valeur minimale comprise entre 780 et 1000 nm, et une valeur maximale comprise entre 1700 et 2500 nm, de préférence ladite variation de la longueur d'onde est comprise entre 900 et 1700 nm.

Selon une mise en oeuvre, on contrôle la température dudit fluide positionné dans ledit milieu transparent pour la réalisation de la mesure spectrale, de préférence en faisant varier la température du fluide entre une température minimale comprise entre -20 °C et 15 °C et une température maximale comprise entre 30 °C et 60 °C.

Selon un mode de réalisation, on fait circuler le fluide dans une conduite, et on réalise ladite mesure spectrale dans une dérivation de ladite conduite, dans laquelle ledit fluide circule.

Selon un aspect, pendant ladite mesure spectrale, on arrête la circulation du fluide dans ladite dérivation.

En outre, l'invention concerne un procédé de surveillance et/ou de contrôle d'un écoulement d'un fluide dans une conduite. Pour ce procédé, on met en oeuvre les étapes suivantes :
a) on détermine au moins un paramètre représentatif d'un changement dudit fluide au sein dudit conduit dû à une variation de température dudit fluide au moyen du procédé selon l'une des caractéristiques précédentes ; et
b) on surveille et/ou on contrôle ledit écoulement dudit fluide dans ledit conduit en fonction dudit paramètre représentatif d'un changement dudit fluide déterminé.

D'autres caractéristiques et avantages du procédé selon l'invention, apparaîtront à la lecture de la description ci-après d'exemples non limitatifs de réalisations, en se référant aux figures annexées et décrites ci-après.

### Liste des figures

[Fig 1]

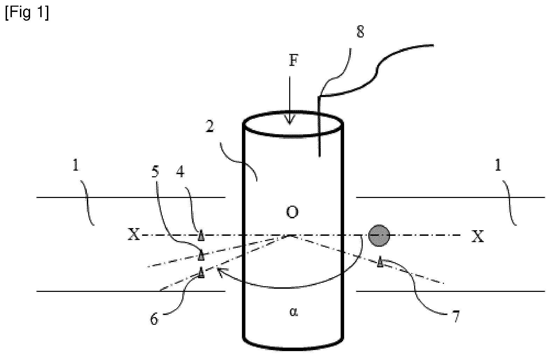

   La figure 1 illustre une sonde SRS et un tube pour la mise en oeuvre du procédé selon un mode de réalisation de l'invention.
[Fig 2]

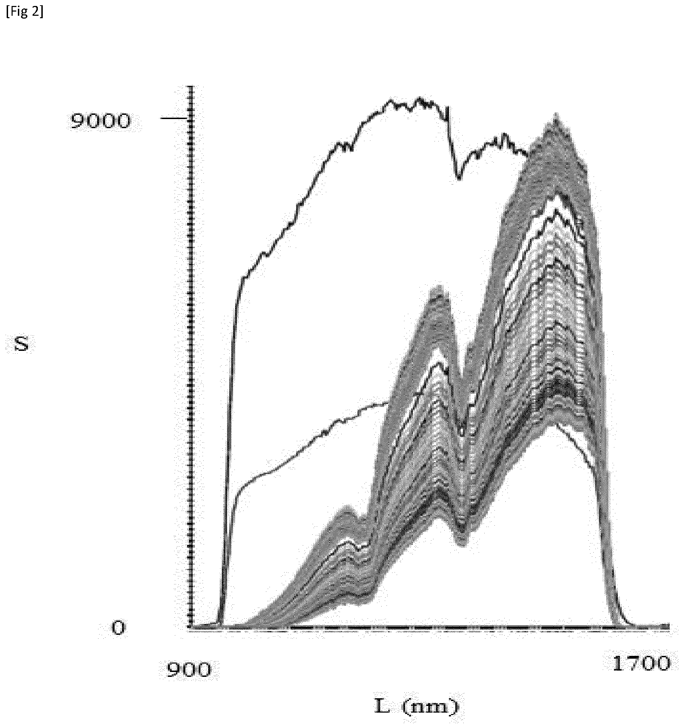

   La figure 2 illustre un ensemble de mesures spectrales de la spectroscopie résolue spatialement pour un échantillon au cours d'un cycle de refroidissement en température pour un exemple selon l'invention.
[Fig 3]

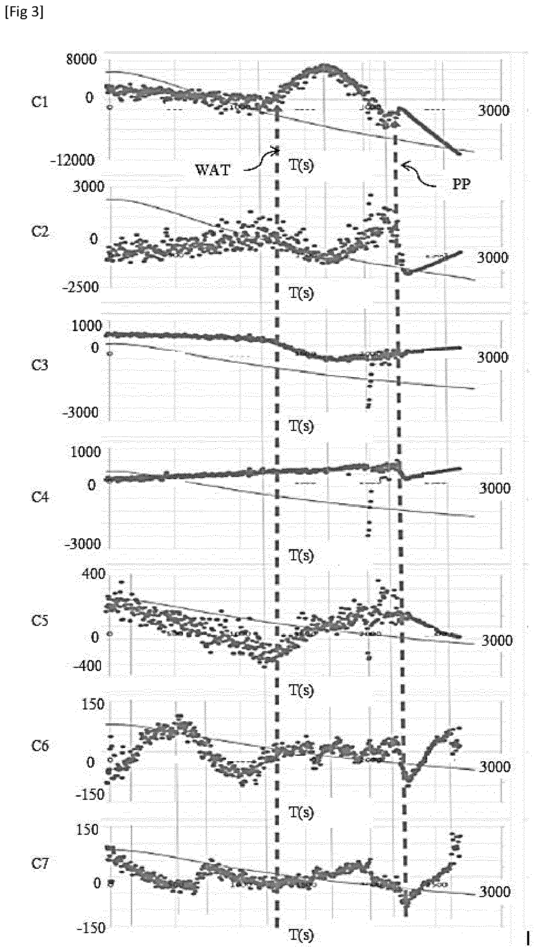

   La figure 3 représente les courbes d'une analyse multivariée pour un exemple selon un mode de réalisation de l'invention.
[Fig 4]

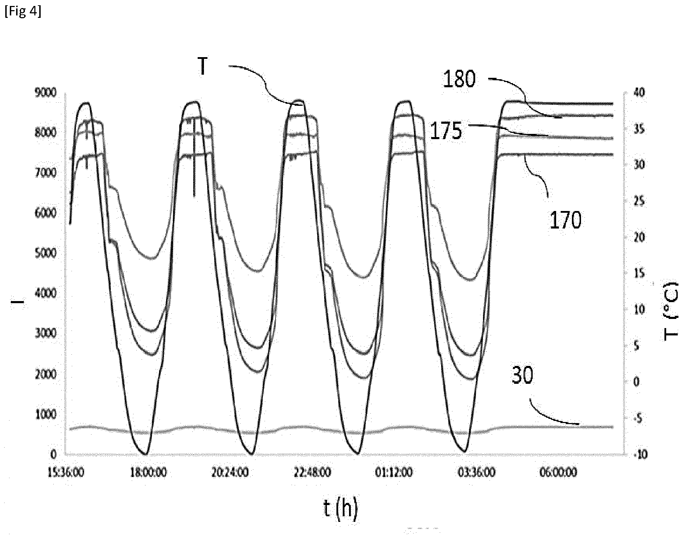

   La figure 4 représente les suivis d'un mélange de bruts de pétrole à une longueur d'onde pour les 4 angles de détection lors de 5 cycles successifs en température pour un exemple selon un mode de réalisation de l'invention.
[Fig 5]

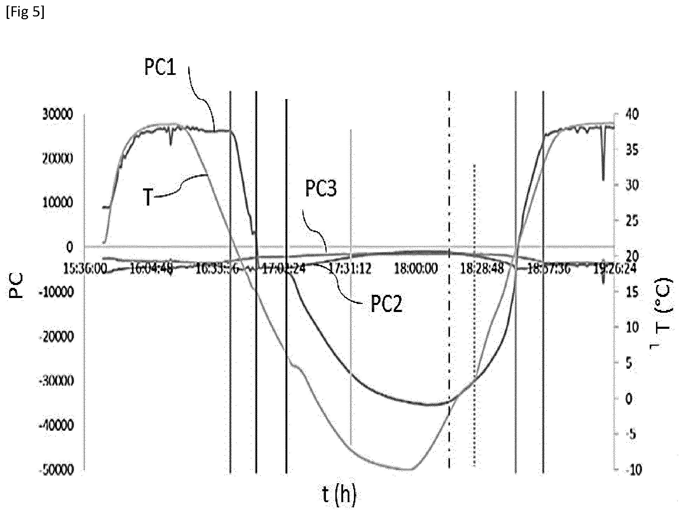

   La figure 5 représente les scores lors d'un cycle en des trois premières composantes principales calculées sur la base de donnée globale pour un exemple selon un mode de réalisation de l'invention
[Fig 6]

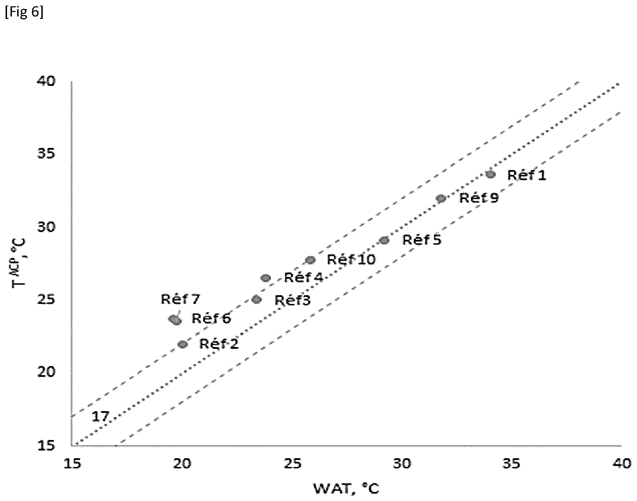

   La figure 6 représente la WAT obtenue par l'analyse des scores de l'ACP en fonction de la WAT obtenue par la méthode de référence pour un exemple selon un mode de réalisation de l'invention.

### Description des modes de réalisation

La présente invention concerne un procédé de détermination, en temps réel, d'au moins un paramètre d'un fluide. Le paramètre est représentatif d'un changement (c'est-à-dire d'une transition ou d'une variation) du fluide lié à une variation de température du fluide. Un tel paramètre est aussi appelé propriété à froid du fluide.

Avantageusement, le paramètre représentatif d'un changement du fluide peut être choisi parmi la température de cristallisation commençante de paraffine WAT dans le fluide, la température du point d'écoulement PP du fluide, le point d'apparition des cristaux, la température limite de filtrabilité, un seuil de transition entre les phases du fluide, un paramètre lié à un agrégat ou une agglomération d'objets au sein du fluide, tel que la génération de nano-agrégats, la croissance des objets, la poly-dispersité des objets, la précipitation des asphaltènes, la cristallisation de l'eau en hydrate, la température de disparition des paraffines (de l'anglais « Wax disappearance température »).

De préférence, le paramètre représentatif d'un changement du fluide peut être la température d'apparition de paraffine WAT dans le fluide et/ou la température du point d'écoulement PP du fluide. De manière préférée, le procédé selon l'invention peut déterminer la WAT et le PP.

Le fluide peut être de tout type, en particulier un fluide organique, par exemple un fluide d'origine pétrolière, de préférence un fluide pétrolier, et de manière très préférée un brut pétrolier, par exemple un brut paraffinique. En effet, le procédé selon l'invention est adaptable à tout type de fluide, et fonctionne pour les fluides opaques comme pour les fluides clairs (transparents, translucides), ou les fluides troubles. Alternativement, le fluide peut être un kérosène ou un distillat.

Le procédé selon l'invention est particulièrement adapté pour déterminer la température d'apparition de paraffine WAT et la température du point d'écoulement PP d'un brut pétrolier.

Pour le mode de réalisation pour lequel le fluide est le kérosène, le paramètre représentatif d'un changement du fluide peut être le point de disparition des cristaux (« freezing point » en anglais). Pour le mode de réalisation pour lequel le fluide est un distillat, le paramètre peut être la température du point d'écoulement, et/ou la température limite de filtrabilité TLF et/ou la température d'apparition des cristaux CP (de l'anglais « cloud point »).

Pour le procédé selon l'invention, on met en oeuvre les étapes suivantes :
a) on dispose un fluide dans un milieu transparent au domaine proche infrarouge ;
b) on réalise une mesure spectrale par spectroscopie proche infrarouge résolue spatialement (PIR - SRS) pour le fluide disposé dans le milieu transparent, la mesure spectrale étant réalisée pour au moins deux angles de mesure et pour au moins deux températures du fluide positionné dans le milieu transparent ;
c) on réalise une analyse multivariée de la mesure spectrale en fonction de la température ; et
d) on détermine le paramètre représentatif du changement du fluide au moyen de l'analyse multivariée.

La spectroscopie PIR-SRS multipoints consiste à mesurer le signal en plusieurs points par rapport à une source d'illumination, qui émet dans le domaine proche infrarouge (c'est-à-dire avec des longueurs d'onde comprises entre 0,78 et 3 µm). Les spectres obtenus peuvent être mesurés en mode réflexion ou dans une configuration qui permet d'acquérir les signaux en réflexion et en transmission. Les mesures SRS associées à des outils d'analyses multivariées (appelés également outils chimiométriques) font ressortir les différents phénomènes présents dans le milieu suivi.

La Spectroscopie Proche Infrarouge (SPIR) offre de nombreux avantages pour le suivi de procédés. Au-delà des aspects pratiques, une des principales raisons de l'utiliser est sa capacité à fournir rapidement et simplement des informations physiques et chimiques. Néanmoins, dans le cas de milieux hétérogènes et/ou complexes, la mesure du signal en un point peut s'avérer insuffisante. Une des solutions pour palier à cela est de mesurer le milieu en plusieurs points, et d'y appliquer des outils d'analyse multivariée. Le procédé selon l'invention permet de réaliser rapidement et simplement des mesures en ligne pour un suivi d'une circulation continue d'un fluide grâce à la spectroscopie proche infrarouge.

La réalisation de la mesure spectrale sur un fluide en écoulement, permet un suivi en continu et en temps réel du paramètre représentatif du changement du fluide. De plus, la réalisation de la mesure pour une pluralité de températures du fluide permet une analyse précise du changement du fluide en fonction de la température.

Pour le procédé selon l'invention, on peut utiliser une sonde SRS Sam-Flex ^{™} (Indatech-Chauvin Arnoux, France).

Selon un aspect de l'invention, le milieu transparent au domaine proche infrarouge peut être un tube transparent. En tant que tube transparent au domaine proche infrarouge, on peut utiliser un tube en quartz. Ce matériau est adapté aux mesures PIR-SRS.

Selon un mode de réalisation de l'invention, on fait circuler le fluide dans une déviation d'un conduit d'écoulement du fluide (ou « pipe » pour la terminologie anglophone dans le domaine des fluides pétroliers). En d'autres termes, la mesure est réalisée dans une branche en parallèle d'un conduit d'écoulement de fluide. Ainsi, il est possible de suivre l'évolution du fluide, notamment en fonction de la température, au sein d'un conduit d'écoulement, en temps réel et en continu au cours d'un cycle de température, et sans ralentir ou stopper l'écoulement principal mais via une boucle rapide alimentée en continue et dont le flux serait stoppé le temps d'un cycle d'analyse en température. Par exemple, le procédé selon l'invention peut permettre de réaliser une analyse en « Stop Flow » dans une dérivation d'une conduite dans laquelle circule le fluide. En d'autres termes, une conduite de circulation du fluide à analyser comprend une dérivation, dans laquelle circule également le fluide, et on réalise la mesure par spectroscopie proche infrarouge dans cette dérivation. Pour réaliser la mesure, on arrête la circulation du fluide uniquement dans la dérivation. Une fois la mesure terminée, on laisse à nouveau circuler le fluide dans la dérivation, et on peut répéter la mesure si on le souhaite. Ainsi, la circulation du fluide dans la conduite n'est pas perturbée et cette méthode ne nécessite pas de prélever un échantillon du fluide.

Afin d'avoir des mesures précises, on peut réaliser la spectroscopie PIR-SRS au moyen d'une pluralité de mesures (en d'autres termes avec une pluralité de points de mesure), selon au moins deux angles de mesure. On appelle angle de mesure l'angle formé entre la direction du signal issu de la source d'illumination et la direction selon laquelle le détecteur reçoit le signal. Lorsqu'on utilise une pluralité de mesures, on parle de spectroscopie multipoints.

Selon un mode de réalisation de l'invention, on peut réaliser une spectroscopie PIR-SRS au moyen d'au moins une mesure en transmission. La mesure en transmission peut être réalisée avec un angle de mesure compris entre 90° et 180° par rapport à la source, de préférence entre 165° et 180°, par exemple avec des valeurs de 170°, 175° ou 180°. Entre 175°et 180°la spectroscopie PIR-SRS est plus sens ible aux variations de diffusion liée aux petites particules.

En sus de la mesure en transmission, on peut réaliser une spectroscopie PIR-SRS au moyen d'au moins une mesure en réflexion, appelée également rétrodiffusion. La mesure en réflexion peut être réalisée avec un angle de mesure compris entre 5° et 90°, de préférence entre 20°et 40°, par exemple avec une valeur de 30 °.

Selon un premier exemple de réalisation de l'invention, on peut réaliser une spectroscopie PIR-SRS au moyen d'au moins deux mesures en transmission avec respectivement des angles de mesure de environ 175°, et environ 180°, et au moyen d'au moins une mesure en réflexion avec un angle de mesure de environ 30°. L'intérêt de cette configuration de la mesure multipoints est d'avoir à la fois des mesures en réflexion et des mesures en transmission pour permettre une mesure spectrale et donc une analyse multivariée précise.

Selon un deuxième exemple de réalisation de l'invention, on peut réaliser une spectroscopie PIR-SRS au moyen de trois mesures en transmission avec respectivement des angles de mesure de environ 170°, environ 175°, et environ 18 0°, et au moyen d'une mesure en réflexion avec un angle de mesure de environ 30°. L'intérêt de cette configuration de la mesure multipoints est d'avoir à la fois des mesures en réflexion et des mesures en transmission pour permettre une mesure spectrale et donc une analyse multivariée précise. L'ajout d'un point de mesure en transmission par rapport au premier exemple de réalisation permet d'avoir un peu plus d'informations sur les différentes tailles de particules du fluide qui diffusent la lumière.

Selon un aspect de l'invention, on peut mettre en oeuvre la spectroscopie proche infrarouge résolue spatialement pour une longueur d'onde de la source de diffusion variant d'une valeur minimale comprise entre 780 et 1000 nm, et une valeur maximale comprise entre 1700 et 3000 nm, de préférence ladite variation de la longueur d'onde est comprise entre 900 et 2200 nm. Ainsi, le procédé selon l'invention balaie une grande plage de longueurs d'onde, ce qui permet d'obtenir des paramètres liés à un changement du fluide, tels que des paramètres liés à la précipitation des asphaltènes ou les paramètres liés à la composition SARA (Saturé Aromatique Résine et Asphaltène) du fluide. En outre, pour augmenter la sensibilité de détection des plus petits objets, il est préférable d'avoir une variation de la longueur d'onde comprenant des petites valeurs (par exemple 800 nm).

### [Fig 1]

La figure 1 illustre, schématiquement et de manière non limitative, une sonde PIR-SRS 1 et un tube 2 pour la mise en oeuvre du procédé selon un mode de réalisation de l'invention. Au sein du tube 2 est positionné un fluide F (le sens de circulation du fluide n'a pas d'importance). Au sein de la sonde PIR-SRS est placée une source d'illumination 3 qui a une direction XX d'illumination. En outre la sonde PIR-SRS comporte quatre détecteurs lumineux 4, 5, 6, et 7. Les détecteurs lumineux 4, 5, 6 et 7 peuvent être déportés : la sonde peut comprendre des fibres optiques (non représentées) qui transmettent les signaux optiques aux détecteurs lumineux 4, 5, 6 et 7. Les détecteurs placés en vis-à-vis de la source d'illumination 3 sont des détecteurs pour des mesures en transmission, et les détecteurs placés du même côté que la source d'illumination sont des détecteurs de mesures en réflexion. Ces quatre détecteurs lumineux sont agencés de la façon suivante : trois en transmission 4, 5 et 6, et un en réflexion 7. Sur cette figure, la direction des signaux lumineux est représentée par des lignes discontinues. Les angles de mesures correspondent aux angles formés par le segment de droite entre la source d'illumination 3 et le point O, avec le segment de droite formé par le point O et le capteur 4, 5, 6, ou 7. Par exemple, l'angle α représente l'angle de mesure du capteur 6. Le capteur 4 (en transmission, dite transmission collimatée) est placé sur l'axe XX, avec un angle de mesure de 180°. Le capteur 5 (en transmission, dite transmission diffuse) est légèrement décalé par rapport au capteur 4, et possède un angle de mesure de 175°. Le capteur 6 (en transmission, dite en transmission diffuse) est légèrement décalé par rapport au capteur 5, et possède un angle de mesure de 170°. Le capteur 7 (en réflexion, appelée également rétrodiffusion) est décalé par rapport à l'axe XX, avec un angle de mesure de 30°.

De plus, de manière optionnelle, le système de mesure peut comprendre une sonde de température 8 pour mesurer la température dans le tube 2.

En outre, selon une mise en oeuvre de l'invention, le système de mesure peut également comprendre un système de régulation de la température (non représenté) du tube 2, de manière à contrôler la température du fluide F, par exemple en diminuant la température du fluide F.

L'étape de traitement des spectres par analyses de données multivariées obtenus par la spectroscopie PIR-SRS permet de différencier les informations contenues dans les spectres. Pour cela, on peut mettre en oeuvre différentes méthodes, telle qu'une analyse en composantes principales (ACP), ou une analyse en composantes communes et poids spécifiques (ACCPS). Ces méthodes permettent une différenciation rapide. Ainsi, il est possible de déterminer le paramètre représentatif d'un changement de fluide en continu. En outre, l'analyse multivariée permet de déterminer au moyen de mesures avec un seul appareil (la spectroscopie proche infrarouge résolue spatialement) une pluralité de paramètres représentatifs d'un changement du fluide. Par exemple, le procédé selon l'invention permet de déterminer avec un seul moyen de mesure la température d'apparition de paraffine et la température du point d'écoulement du fluide.

L'ACP est un outil d'analyse de données multivariées permettant d'explorer un jeu de données ayant un grand nombre de variables. L'ACP consiste à représenter les individus (échantillons) dans un espace plus réduit défini par les variables. C'est une méthode qui vise à trouver les directions de plus grande dispersion des individus dans cet espace, l'idée étant que les directions de plus grande dispersion sont les directions les plus intéressantes. Si les variables ne contiennent que du bruit, les individus seront dispersés de façon homogène et uniforme dans toutes les directions. Une direction qui s'écarte d'une telle répartition sphérique peut potentiellement contenir de l'information. Mathématiquement, l'ACP calcule des combinaisons linéaires des variables de départ donnant de nouveaux axes, appelés composantes principales (CP), qui contiennent la plus grande partie de la variabilité de la matrice de données de départ. Elle fait simplement l'hypothèse, que les directions de plus grandes dispersions des échantillons sont les directions les plus intéressantes et que la variabilité associée avec ces directions correspond à de l'information. De plus, pour éviter d'avoir la même «information» dans plusieurs Composantes Principales, elles doivent toutes être orthogonales. La décomposition matricielle de l'ACP permet d'obtenir des matrices des coordonnées factorielles (ou «scores») et des contributions factorielles (ou «loadings»).

L'ACCPS est un outil d'analyse multivariée qui permet d'analyser simultanément plusieurs matrices de données, considérées comme des tableaux, et d'extraire l'information commune entres elles. Initialement, cette méthode a été développée par Qannari et al. (décrite par exemple dans le document Hanafi M, Qannari EM. Nouvelles propriétés de l'analyse en composantes communes et poids spécifiques. Journal de la Société Française de Statistique 2008;149(2)) pour analyser des données issues d'études sensorielles. Plus récemment, elle a été utilisée pour étudier des échantillons mesurés avec différents instruments, pour trouver des corrélations entre des tableaux de données et pour discriminer des échantillons en utilisant l'information globale contenue dans chaque tableau.

Conformément à une mise en oeuvre de l'invention, pour l'analyse multivariée de type ACP ou ACCPS, on peut déterminer au moins six composantes, de manière à obtenir une information précise et pour avoir la possibilité d'obtenir une pluralité de paramètres liés à un changement du fluide.

Selon un mode de réalisation de l'invention, on peut déterminer le paramètre représentatif d'un changement du fluide par analyse d'au moins un point d'inflexion et/ou une rupture de pente et/ou la limitation du bruit du signal d'au moins une composante de l'analyse multivariée. En effet, les points d'inflexions et/ou les ruptures de pente et/ou la présence de bruit des composantes de l'analyse multivariée indiquent des variations/changements dans le fluide.

Conformément à une mise en oeuvre de l'invention, on peut contrôler la température du fluide circulant dans le milieu transparent pour la réalisation de la mesure spectrale. Pour cette mise en oeuvre, on peut faire varier la température du fluide entre une température minimale comprise entre -20 °C et 15°C et une température ma ximale comprise entre 30 °C et 60 °C. Ainsi, il est possible d'analyser précisé ment le(s) changement(s) du fluide et d'obtenir le paramètre recherché.

Selon un aspect de l'invention, le procédé peut comporter également une étape de mesure de la température du fluide dans le milieu transparent. Cette étape de mesure de la température peut être mise en oeuvre au moyen d'une sonde de température dans le milieu transparent.

En outre, la présente invention, concerne un procédé de surveillance et/ou de contrôle d'un écoulement d'un fluide dans une conduite (par exemple d'un brut pétrolier dans un « pipe »). Dans ce cas, on met en oeuvre les étapes suivantes :
a) on détermine au moins un paramètre représentatif d'un changement du fluide au sein de la conduite dû à une variation de température du fluide au moyen du procédé selon l'une quelconque des variantes décrites précédemment ; et
b) on surveille et/ou on contrôle l'écoulement du fluide dans la conduite en fonction du paramètre représentatif d'un changement du fluide déterminé à l'étape précédente.

Selon un mode de réalisation de l'invention, dans le cadre de l'écoulement d'un brut pétrolier dans un « pipe », on peut déterminer lors de l'étape a) la température d'apparition de paraffine WAT et/ou la température du point d'écoulement PP du brut pétrolier, la présence de particules et/ou d'un encrassement. En effet, les paraffines peuvent, en-dessous de la WAT, former des dépôts sur les parois internes de la conduite de transport et conduite à une obstruction de la conduite qui génère soit une forte de perte de charge, soit un bouchage de la conduite. Ce même brut paraffinique peut gélifier en masse, en particulier lors d'une phase d'arrêt et nécessiter pour son redémarrage des pressions importantes. La détermination de la WAT et du « Pour Point » PP pour ce type de fluide pétrolier est très importante pour anticiper les architectures de champs et les opérations de remédiation.

Cependant, la composition du fluide peut varier pour diverses raisons (ligne d'export transportant un mélange d'huiles en proportions variables, évolution de la composition du pétrole brut produit au cours de la durée d'exploitation du champ). Cette variation de composition induit également des variations des valeurs de WAT et PP du fluide transporté.

La surveillance et/ou le contrôle peut consister en au moins l'une des étapes suivantes :
- optimisation des additifs dans le fluide, par exemple des additifs antigel ou des additifs d'élimination des bouchages de conduite,
- la surveillance de l'apparition d'asphaltènes floculés,
- la détection de phénomènes liés à l'instabilité des produits,
- optimisation de la stratégie de production, par exemple, dans le cadre de l'écoulement d'un brut pétrolier dans un « pipe », il est possible de déterminer une durée maximale admissible pour un arrêt de la production, pendant lequel le fluide se refroidit, ou la nécessité de recourir à la mise en oeuvre d'un chauffage de la conduite, ou la détermination de la fréquence des opérations de raclage de la conduite.

Grâce au procédé selon l'invention, l'exploitant a accès à une mesure en temps réel de WAT et PP lui permettant de mieux apprécier la marge de sécurité et ainsi optimiser ses stratégies de production.

### Exemples

Les caractéristiques et avantages du procédé selon l'invention apparaîtront plus clairement à la lecture de l'exemple d'application ci-après.

Afin d'illustrer cette invention, des essais ont été réalisés sur un brut pétrolier en faisant varier la température pour suivre le comportement entre l'apparition de paraffine (à la WAT) et l'état figé du système (température du point d'écoulement PP).

Dans le cas des essais, une sonde multipoint, Sam-Flex ^{™}, Indatech-Chauvin Arnoux, a été utilisée. Dans cet exemple, les mesures ont été faites avec des angles de mesure de 180°, 175°, 170° et 30° permettant de mesurer le signal à la fois en réflexion et en transmission comme sur la Figure 1.

Dans la configuration utilisée l'entrefer de la sonde est de 3 mm, il équivaut au trajet optique.

La sonde a été reliée à un spectromètre équipé d'une détection hyperspectrale pour pouvoir mesurer les spectres aux quatre angles en même temps (Hy-ternity ^{™}, Indatech-Chauvin Arnoux). Le détecteur InGaAs a permis de réaliser des mesures dans la gamme spectrale allant de 900 à 1700 nm.

L'échantillon analysé était un pétrole brut dont la WAT et le PP (initialement connues) sont séparés de 3°C.

L'échantillon a été mis sous agitation dans un bain marie pour couvrir une gamme de température allant de WAT + 5°C à PP -5°C. Les mesu res spectrales ont été faites à la fois lors de la montée en température et lors de la descente en température. Le temps d'intégration était de 6s toutes les 5s.

### [Fig 2]

La figure 2 illustre le signal S (sans unité) correspondant à l'intensité brute reçue sur le détecteur en fonction de la longueur d'onde L en nm obtenu pour l'angle de mesure de 180°. Des courbes similaires sont obtenues pour les autres angles de mesure (non représentées). Les différentes courbes correspondent aux spectres obtenues pour les différentes températures. Les variations d'intensité observées sur l'axe des ordonnées sont causées par les modifications du milieu analysé, elles même dues à la modification de température.

Sur cette courbe, les premier creux (vers 1200nm) correspondent à la deuxième harmonique des élongations symétriques et asymétriques des groupements CH₂ et CH₃, tandis que vers 1400 nm, il s'agit de la première harmonique de la combinaison des bandes classiquement vers 4000cm⁻¹ (combinaison entre les élongations symétriques et asymétriques des liaisons CH dans les groupements CH₂ et CH₃ et les déformations des liaisons C-H).

Pour une exploitation multivariée des données, une analyse en composantes principales ACP a été réalisée. Il aurait également été possible de réaliser une analyse ne composantes communes et poids spécifiques ACCPS, mais ici les données ont été exploitées plus simplement après dépliement en 2D.

L'objectif est de représenter sur un nombre limité de composantes, l'essentiel de l'information SRS au cours du temps. De l'information pertinente a pu être observée jusqu'à minima la sixième composante principale.

Les composantes ACP sont orthogonales par construction c'est à dire que l'information représentée sur chaque axe est indépendante. Les scores obtenus jusqu'à la septième composante sont représentés à la figure 3.

### [Fig 3]

La figure 3 représente les sept composantes principales C1 à C7 en fonction du temps T en s. Sur cette figure les points correspondent aux scores (valeurs pour la composante principale considérée) en fonction du temps T et la courbe correspond à la température du milieu en fonction du temps T.

Sur ces composantes, cinq ruptures de pentes ont été observées, dont deux correspondant à la température d'apparition de paraffine WAT et à la température du point d'écoulement du fluide PP:
- la WAT est décrite par la première flèche verticale en traits pointillés (à gauche) et se situe au niveau d'une première rupture de pente sur les composantes C1, C2, C3 et C5, et
- le PP, décrite par la deuxième flèche verticale en traits pointillés, coïncide naturellement avec le changement attendu du bruit de mesure du signal : lorsque le produit passe d'un mode en circulation dynamique : signal assez bruité ; à un signal beaucoup plus propre lié à une acquisition en mode statique, sur l'ensemble des composantes.

Les autres ruptures de pente des composantes traduisent d'autres changements de propriétés du fluide.

Ainsi, le procédé selon l'invention permet de déterminer précisément des paramètres de changement d'un fluide, tels que la WAT et le PP.

Pour compléter l'exemple précédent, des essais complémentaires ont été réalisés et sont présentés ci-après :
Le matériel de mesure spectrale, sonde et spectromètre, ainsi que la façon de procéder sont les mêmes que dans l'exemple précédent. Toutefois, les cycles en température vont pour cet exemple de 40 °C à -20 °C et ceci est répété da ns un soucis de valider la répétabilité des essais.

Pour les essais ci-après, trois bruts pétroliers ont été utilisés (fractions A , B et C), pur ou en mélange, afin d'obtenir 10 références échantillons différentes. Les proportions ainsi que les WAT des références échantillons sont présentées dans le tableau suivant :

**[Table 1]**

| **Réf. échantillon** | **Fraction A** | **Fraction B** | **Fraction C** | WAT (°C) |
|---|---|---|---|---|
| 1 | 100% | 0 | 0 | 34 |
| 2 | 0 | 100% | 0 | 20 |
| 3 | 0 | 0 | 100% | 23,4 |
| 4 | 30% | 70% | 0 | 23,8 |
| 5 | 70% | 30% | 0 | 29,2 |
| 6 | 0 | 30% | 70% | 19,6 |
| 7 | 0 | 70% | 30% | 19,7 |
| 8 | 30% | 0 | 70% | 29,5 |
| 9 | 70% | 0 | 30% | 31,8 |
| 10 | 34% | 33% | 33% | 25,8 |

Pour chaque échantillon les mêmes cycles en température ont été réalisés. Les spectres sont acquis toutes les minutes avec un temps d'intégration de 90 ms.

Pour valider la répétabilité et la fiabilité des essais, les mesures des cycles en température réalisé sur un échantillon ont été représentées sur la figure 4.

### [Fig 4]

La figure 4 représente les suivis à 1555 nm pour les 4 angles de l'échantillon 7 lors de 4 cycles en température. Soit quatre descentes en température de 40 à -20°C et 4 montées en température de -20 à 40 °C. La figure montre que les intensités I mesurées aux quatre angles (30°, 170°, 175°, 180°) en fonction du temp **stenh** sont très similaires d'un cycle à un autre. De plus, la figure 4 illustre la courbe de la température T en °C en fonction du temps t en h. Cela prouve que les expérimentations sont bien répétables, ce qui valide le protocole opératoire. Ainsi l'exploitation des résultats peut être faite sur un seul cycle de température mais reste valable à chaque fois.

Comme dans l'exemple précédent, une analyse multivariée des données est réalisée. Ainsi, une ACP des spectres d'un cycle en température de tous les échantillons est mise en oeuvre. Ensuite, les mesures d'un cycle en température de chaque échantillon sont projetées alternativement sur cette ACP globale pour l'exploitation des résultats. Cela permet de tracer pour chaque échantillon les scores des trois premières composantes principales, et de relier chaque rupture de pente à sa température. Un exemple de représentation des scores est présenté sur la figure 5 pour l'échantillon 7.

### [Fig5]

Sur la figure 5 les scores des trois premières composantes principales (PC1, PC2, PC3) en fonction du temps t en h lors d'un cycle en température de l'échantillon 7 ont été tracés. De plus, la figure 4 illustre la courbe de la température T en °C en fonction du temps t en h. Sur cette figure des traits verticaux sont ajoutés lorsqu'un changement de pente a été observé sur les scores. Cette démarche a été appliquée pour tous les échantillons. L'exploitation de ces résultats montre que la première rupture de pente est liée à la WAT des échantillons.

### [Fig 6]

La WAT obtenue par l'étude des scores de l'ACP (T^{ACP} en °C) a été tracée en fonction de celle mesurée par la méthode de référence (DSC = Differential Scanning Calorimetry) WAT en °C sur la figure 6, pour chacun des échantillons réf1 à réf 10.

Une dispersion quasi linéaire des échantillons est observée sur la figure 6..

Cet exemple, avec de nouveaux bruts pétrolier, valide le fait que le procédé selon l'invention permet de déterminer précisément des paramètres de changement d'un fluide tel que la WAT.

## Revendications

1. Procédé de détermination d'au moins un paramètre représentatif d'un changement d'un fluide dû à une variation de température dudit fluide, dans lequel on met en oeuvre les étapes suivantes :
a) on dispose ledit fluide (F) dans un milieu transparent (2) au domaine proche infrarouge ;
b) on réalise une mesure spectrale par spectroscopie proche infrarouge résolue spatialement pour ledit fluide (F) disposé dans ledit milieu transparent (2), ladite mesure spectrale étant réalisée pour au moins deux angles de mesure et pour au moins deux températures dudit fluide ;
c) on réalise une analyse multivariée de ladite mesure spectrale en fonction de ladite température ; et
d) on détermine ledit paramètre représentatif d'un changement dudit fluide au moyen de ladite analyse multivariée.

2. Procédé selon la revendication 1, dans lequel ledit paramètre représentatif d'un changement dudit fluide est choisi parmi la température d'apparition de paraffine dans ledit fluide, la température du point d'écoulement dudit fluide, un point d'apparition des cristaux, une température limite de filtrabilité, un seuil de transition entre les phases dudit fluide, un paramètre lié à un agrégat ou une agglomération d'objets au sein dudit fluide, tel que la génération de nano-agrégats, la croissance des objets, la poly-dispersité des objets.

3. Procédé selon la revendication 2, dans lequel ledit paramètre représentatif d'un changement dudit fluide est la température d'apparition de paraffine dans ledit fluide et/ou la température du point d'écoulement dudit fluide.

4. Procédé selon l'une des revendications précédentes, dans lequel ledit fluide est un brut pétrolier.

5. Procédé selon l'une des revendications précédentes, dans lequel on dispose ledit fluide dans une déviation d'un conduit d'écoulement dudit fluide.

6. Procédé selon l'une des revendications précédentes, dans lequel on réalise ladite spectroscopie proche infrarouge résolue spatialement au moyen d'au moins une mesure en transmission, notamment avec un angle de mesure (α) compris entre 130°et 180°, de préférence entre 165° et 180°.

7. Procédé selon la revendication 6, dans lequel on réalise ladite spectroscopie proche infrarouge résolue spatialement au moyen d'au moins une mesure en réflexion, notamment avec un angle de mesure (α) compris entre 5° et 90°, de préférence entre 20 et 40°.

8. Procédé selon la revendication 7, dans lequel on réalise ladite spectroscopie proche infrarouge résolue spatialement au moyen d'au moins deux mesures (4, 5, 6) en transmission avec respectivement des angles de mesure (α) choisis parmi environ 170°, 175°et 180°et au moyen d'une mesure (7) en réflex ion avec un angle de mesure (α) de environ 30°.

9. Procédé selon l'une des revendications précédentes, dans lequel ladite analyse multivariée est une analyse en composantes principales ou une analyse en composantes communes et poids spécifiques.

10. Procédé selon la revendication 9, dans lequel ladite analyse multivariée est réalisée pour au moins six composantes.

11. Procédé selon l'une des revendications 9 ou 10, dans lequel on détermine ledit paramètre représentatif d'un changement dudit fluide par analyse d'au moins un point d'inflexion et/ou une rupture de pente et/ou la limitation du bruit d'au moins une composante de ladite analyse multivariée.

12. Procédé selon l'une des revendications précédentes, dans lequel ladite spectroscopie proche infrarouge résolue spatialement est mise en oeuvre avec variation de la longueur d'onde entre une valeur minimale comprise entre 780 et 1000 nm, et une valeur maximale comprise entre 1700 et 2500 nm, de préférence ladite variation de la longueur d'onde est comprise entre 900 et 1700 nm.

13. Procédé selon l'une des revendications précédentes, dans lequel on contrôle la température dudit fluide positionné dans ledit milieu transparent pour la réalisation de la mesure spectrale, de préférence en faisant varier la température du fluide entre une température minimale comprise entre -20 °C et 15 °C et une température maximale comprise entre 30 °C et 60 °C.

14. Procédé selon l'une des revendications précédentes, dans lequel on fait circuler le fluide dans une conduite, et on réalise ladite mesure spectrale dans une dérivation de ladite conduite, dans laquelle ledit fluide circule.

15. Procédé selon la revendication 14, dans lequel, pendant ladite mesure spectrale, on arrête la circulation du fluide dans ladite dérivation.

16. Procédé de surveillance et/ou de contrôle d'un écoulement d'un fluide dans une conduite, **caractérisé en ce qu'**on met en oeuvre les étapes suivantes :
a) on détermine au moins un paramètre représentatif d'un changement dudit fluide au sein dudit conduit dû à une variation de température dudit fluide au moyen du procédé selon l'une des revendications précédentes ; et
b) on surveille et/ou on contrôle ledit écoulement dudit fluide dans ledit conduit en fonction dudit paramètre représentatif d'un changement dudit fluide déterminé.

## Patentansprüche

1. Verfahren zur Bestimmung mindestens eines für eine Veränderung eines Fluids, die durch eine Temperaturänderung des Fluids bedingt ist, repräsentativen Parameters, bei dem man die folgenden Schritte durchführt:
a) man ordnet das Fluid (F) in einem für den Nahinfrarotbereich (2) transparenten Milieu an;
b) man führt eine spektrale Messung durch räumlich aufgelöste Nahinfrarot-Spektroskopie für das in dem transparenten Milieu (2) angeordnete Fluid (F) aus, wobei die spektrale Messung für mindestens zwei Messwinkel und für mindestens zwei Temperaturen des Fluids ausgeführt wird;
c) man führt eine multivariate Analyse der spektralen Messung in Abhängigkeit von der Temperatur aus; und
d) man bestimmt den für eine Veränderung des Fluids repräsentativen Parameter mittels der multivariaten Analyse.

2. Verfahren nach Anspruch 1, bei dem der für eine Veränderung des Fluids repräsentative Parameter unter der Wachserscheinungstemperatur in dem Fluid, dem Pourpoint des Fluids, dem Cloudpoint, einem Filtrierbarkeitstemperaturgrenzwert, einem Übergangsschwellenwert zwischen den Phasen des Fluids, einem Parameter, der mit einem Aggregat oder einer Agglomeration von Objekten in dem Fluid wie der Erzeugung von Nanoaggregaten, dem Wachstum der Objekte, der Polydispersität der Objekte zusammenhängt, gewählt ist.

3. Verfahren nach Anspruch 2, bei dem der für eine Veränderung des Fluids repräsentative Parameter die Wachserscheinungstemperatur in dem Fluid und/oder der Pourpoint des Fluids ist.

4. Verfahren nach einem der vorhergehenden Ansprüche, bei dem das Fluid ein Rohöl ist.

5. Verfahren nach einem der vorhergehenden Ansprüche, bei dem man das Fluid in einer Abzweigung eines Strömungskanals des Fluids anordnet.

6. Verfahren nach einem der vorhergehenden Ansprüche, bei dem man die räumlich aufgelöste Nahinfrarot-Spektroskopie mittels mindestens einer Messung im Durchlicht ausführt, insbesondere mit einem Messwinkel (α) zwischen 130° und 180°, bevorzugt zwischen 165° und 180°.

7. Verfahren nach Anspruch 6, bei dem man die räumlich aufgelöste Nahinfrarot-Spektroskopie mittels mindestens einer Messung im Auflicht ausführt, insbesondere mit einem Messwinkel (α) zwischen 5° und 90°, bevorzugt zwischen 20 und 40°.

8. Verfahren nach Anspruch 7, bei dem man die räumlich aufgelöste Nahinfrarot-Spektroskopie mittels von mindestens zwei Messungen (4, 5, 6) im Durchlicht mit jeweils Messwinkeln (α), die zwischen etwa 170°, 175° und 180° gewählt sind, und mittels einer Messung (7) im Auflicht mit einem Messwinkel (α) von etwa 30° ausführt.

9. Verfahren nach einem der vorhergehenden Ansprüche, bei dem die multivariate Analyse eine Hauptkomponentenanalyse oder eine Analyse nach gemeinsamen Komponenten und spezifischen Gewichten ist.

10. Verfahren nach Anspruch 9, bei dem die multivariate Analyse für mindestens sechs Komponenten ausgeführt wird.

11. Verfahren nach einem der Ansprüche 9 oder 10, bei dem man den für eine Veränderung des Fluids repräsentativen Parameter durch Analyse mindestens eines Wendepunkts und/oder eines Neigungsbruchs und/oder die Beschränkung des Rauschens mindestens einer Komponente der multivariaten Analyse bestimmt.

12. Verfahren nach einem der vorhergehenden Ansprüche, bei dem die räumlich aufgelöste Nahinfrarot-Spektroskopie mit Änderung der Wellenlänge zwischen einem Mindestwert zwischen 780 und 1000 nm und einem Höchstwert zwischen 1700 und 2500 nm durchgeführt wird, wobei die Änderung der Wellenlänge bevorzugt zwischen 900 und 1700 nm liegt.

13. Verfahren nach einem der vorhergehenden Ansprüche, bei dem man die Temperatur des zur Ausführung der spektralen Messung in dem transparenten Milieu positionierten Fluids kontrolliert, bevorzugt, indem man die Temperatur des Fluids zwischen einer Mindesttemperatur zwischen -20 °C und 15 °C und einer Höchsttemperatur zwischen 30 °C und 60 °C ändert.

14. Verfahren nach einem der vorhergehenden Ansprüche, bei dem man das Fluid in einer Leitung zirkulieren lässt und man die spektrale Messung in einem Bypass der Leitung, in dem das Fluid zirkuliert, ausführt.

15. Verfahren nach Anspruch 14, bei dem man während der spektralen Messung die Zirkulation des Fluids in dem Bypass stoppt.

16. Verfahren zur Überwachung und/oder Kontrolle eines Strömens eines Fluids in einer Leitung, **dadurch gekennzeichnet, dass** man die folgenden Schritte durchführt:
a) man bestimmt mindestens einen für eine Veränderung des Fluids in dem Kanal, die durch eine Temperaturänderung des Fluids bedingt ist, repräsentativen Parameter mittels des Verfahrens nach einem der vorhergehenden Ansprüche; und
b) man überwacht und/oder man kontrolliert das Strömen des Fluids in dem Kanal in Abhängigkeit von dem bestimmten Parameter, der für eine Veränderung des Fluids repräsentativ ist.

## Claims

1. Method for the determination of at least one parameter representative of a change in a fluid due to a variation in temperature of said fluid, in which the following stages are carried out:
a) said fluid (F) is placed in a medium (2) transparent to the near infrared region;
b) a spectral measurement by spatially resolved near infrared spectroscopy is carried out for said fluid (F) placed in said transparent medium (2), said spectral measurement being carried out for at least two measurement angles and for at least two temperatures of said fluid;
c) a multivariate analysis of said spectral measurement as a function of said temperature is carried out; and
d) said parameter representative of a change in said fluid is determined by means of said multivariate analysis.

2. Method according to Claim 1, in which said parameter representative of a change in said fluid is chosen from the wax appearance temperature in said fluid, the temperature of the pour point of said fluid, a point of appearance of the crystals, a cold filter plugging point, a transition threshold between the phases of said fluid, a parameter related to an aggregate or an agglomeration of objects within said fluid, such as the generation of nanoaggregates, the growth of the objects, the polydispersity of the objects.

3. Method according to Claim 2, in which said parameter representative of a change in said fluid is the wax appearance temperature in said fluid and/or the temperature of the pour point of said fluid.

4. Method according to one of the preceding claims, in which said fluid is a crude oil.

5. Method according to one of the preceding claims, in which said fluid is placed in a bypass of a flow pipe for said fluid.

6. Method according to one of the preceding claims, in which said spatially resolved near infrared spectroscopy is carried out by means of at least one measurement in transmission, in particular with a measurement angle (α) of between 130° and 180°, preferably between 165° and 180°.

7. Method according to Claim 6, in which said spatially resolved near infrared spectroscopy is carried out by means of at least one measurement in reflection, in particular with a measurement angle (α) of between 5° and 90°, preferably between 20° and 40°.

8. Method according to Claim 7, in which said spatially resolved near infrared spectroscopy is carried out by means of at least two measurements (4, 5, 6) in transmission with respectively measurement angles (α) chosen from approximately 170°, 175° and 180° and by means of a measurement (7) in reflection with a measurement angle (α) of approximately 30°.

9. Method according to one of the preceding claims, in which said multivariate analysis is a principal components analysis or a common components and specific weights analysis.

10. Method according to Claim 9, in which said multivariate analysis is carried out for at least six components.

11. Method according to either of Claims 9 and 10, in which said parameter representative of a change in said fluid is determined by analysis of at least one point of inflection and/or one break in slope and/or the limitation of the noise of at least one component of said multivariate analysis.

12. Method according to one of the preceding claims, in which said spatially resolved near infrared spectroscopy is carried out with variation in the wavelength between a minimum value of between 780 and 1000 nm and a maximum value of between 1700 and 2500 nm, preferably said variation in the wavelength being between 900 and 1700 nm.

13. Method according to one of the preceding claims, in which the temperature of said fluid positioned in said transparent medium for carrying out the spectral measurement is controlled, preferably by varying the temperature of the fluid between a minimum temperature of between -20°C and 15°C and a maximum temperature of between 30°C and 60°C.

14. Method according to one of the preceding claims, in which the fluid is circulated in a pipe and said spectral measurement is carried out in a bypass of said pipe, in which said fluid circulates.

15. Method according to Claim 14, in which, during said spectral measurement, the circulation of the fluid in said bypass is halted.

16. Method for monitoring and/or controlling a flow of a fluid in a pipe, **characterized in that** the following stages are carried out:
a) at least one parameter representative of a change in said fluid within said pipe due to a variation in temperature of said fluid is determined by means of the method according to one of the preceding claims; and
b) said flow of said fluid in said pipe is monitored and/or controlled as a function of said determined parameter representative of a change in said fluid.
